# FASCICULE DE BREVET EUROPEEN

(11) **EP 3 386 496 B1**
(45) Date de publication et mention de la délivrance du brevet: **11.12.2019**
(21) Numéro de dépôt: 16778078.2
(22) Date de dépôt: 26.09.2016
(51) Int. Cl.: A61K 31/19, A61K 31/728, A61P 19/02

(54) **NOUVELLE COMPOSITION INJECTABLE; PROCEDE DE PREPARATION DE LADITE COMPOSITION; UTILISATION DE LADITE COMPOSITION**
NEUARTIGE INJIZIERBARE ZUSAMMENSETZUNG, VERFAHREN ZUR HERSTELLUNG DER BESAGTEN ZUSAMMENSETZUNG, VERWENDUNG DER BESAGTEN ZUSAMMENSETZUNG
NOVEL INJECTABLE COMPOSITION; METHOD FOR PREPARING SAID COMPOSITION; USE OF SAID COMPOSITION

(30) Priorité: 07.12.2015 FR 1502545
(43) Date de publication de la demande: 17.10.2018
(73) Titulaire: KH Medtech Sàrl, 1204 Genève (CH)
(72) Inventeur: TAUZIN, Bénédicte Vincente, 74250 Bogeve (FR)
(74) Mandataire: reuteler & cie SA
(86) Numéro de dépôt international: PCT/FR2016/000151
(87) Numéro de publication internationale: WO 2017/098091

(56) Documents cités:
- EP-A1- 0 480 189
- EP-A1- 0 499 164
- WO-A2-02/39977

## Description

La présente invention a pour objet :
- Une nouvelle composition aqueuse stérile injectable, stérilisée à la chaleur, comprenant au moins de l'acide hyaluronique réticulé, ou l'un de ses sels, et un ou plusieurs acides gras, caractérisée en ce que :
   ∘ la proportion massique en eau est supérieure à 51% de la masse totale
   ∘ la proportion massique en acide gras est inférieure à 45% de la masse totale
   ∘ les propriétés de viscoélasticité sont telles que le rapport G"/G' à 0.7 Hz est inférieur à 0.70
- Un procédé de préparation de ladite composition
- L'utilisation de ladite composition pour des applications esthétiques et thérapeutiques

L'acide hyaluronique est un polysaccharide formé par la répétition d'une unité disaccharidique composée d'acide D-glucuronique et de N-acétylglucosamine. Sa structure est linéaire et sans spécificité d'espèce. L'acide hyaluronique est largement distribué dans les organismes humains et animaux, dans lesquels il joue de nombreuses fonctions biologiques comme par exemple le contrôle du taux d'hydratation ou le maintien de la viscoélasticité de fluides ou de tissus. On le retrouve notamment en concentration élevée dans le liquide synovial, le corps vitreux de l'œil et dans la peau. Un être humain de 70 kg possède environ 15 g d'acide hyaluronique dont la moitié est contenue dans la peau et cette quantité diminue avec le vieillissement.

Les hydrogels d'acide hyaluronique sont connus et utilisés dans de larges domaines de l'esthétique et de la médecine depuis de nombreuses années. Ces gels sont, notamment, couramment injectés :
- dans les yeux, au cours de chirurgies ophtalmologiques, afin de maintenir l'espace intra-oculaire et protéger les tissus de l'œil
- dans les articulations, en cas d'arthrose, pour supplémenter le liquide synovial déficient et restaurer temporairement les propriétés chondroprotectrices dudit liquide biologique
- dans ou sous la peau, afin de combler des rides ou d'augmenter le volume de certaines zones du visage ou du corps

L'acide hyaluronique possède une demi-vie faible dans les organismes vivants (moins de 1 semaine).

Dans de nombreuses applications en esthétique et en médecine, il est injecté chez les patients sous sa forme native, c'est-à-dire qu'il n'est pas réticulé et/ou modifié chimiquement.

Pour d'autres applications, il est administré chez les patients sous une forme stabilisée par réticulation. La réticulation permet de considérablement augmenter la durée de vie (encore appelée rémanence) de l'acide hyaluronique *in vivo,* mais elle permet également de modifier ses propriétés biomécaniques / rhéologiques en le rendant notamment plus élastique, ce qui permet alors d'accroître sa capacité à créer du volume une fois injecté dans les tissus souhaités.

Des études récentes (par exemple, Farkas et al, Plast Reconst Surg Glob Open, 2013, Mai 7, 1-8) ont permis de mettre en évidence que le vieillissement implique des pertes importantes de volume, en grande partie liées à la fonte des compartiments graisseux, au niveau du visage mais également au niveau du corps.

Ces pertes de volume sont également observées dans le cas de maladies, comme par exemple dans le cas du VIH pour lequel les traitements par antirétroviraux induisent des lipodystrophies pouvant être extrêmement sévères.

Que se soit dans le cas du traitement du vieillissement ou dans le cas du traitement de maladies, les solutions actuelles les plus couramment utilisées pour rétablir et/ou augmenter les volumes du visage ou du corps sont les suivantes :
- Injection de graisse autologue dans les zones anatomiques souhaitées. Cette technique a pour avantage de fournir des résultats long terme (potentiellement quasi permanents) mais elle requiert de faire appel à la chirurgie (acte invasif nécessitant de prélever de la graisse au niveau d'une zone du corps avant de la réimplanter dans une autre zone) et une fraction importante de cette graisse ne résiste pas au transfert réalisé (en général, une perte de volume de l'ordre de 20 à 40% est observée dans les mois qui suivent la chirurgie)
- Injection de produits de comblement, en particulier à base d'acide hyaluronique réticulé. Cette technique, non invasive et rapide à mettre en œuvre, permet de combler des volumes sur des périodes allant de 3 mois à plus de 18 mois en fonction des zones traitées et des produits sélectionnés

L'acide hyaluronique réticulé joue donc un rôle clé dans les domaines de l'esthétique et de la médecine et c'est pour cette raison que l'homme de l'art cherche constamment à améliorer la performance de cette molécule en lui octroyant de meilleures caractéristiques biomécaniques et/ou de rémanence, tout en lui conservant un très haut niveau de sécurité.

La présente invention vise précisément à proposer une nouvelle composition aqueuse stérile injectable, stérilisée à la chaleur, contenant au moins de l'acide hyaluronique réticulé, ou l'un de ses sels, et un ou plusieurs acides gras; composition sous forme de gel qui permet notamment :
- d'accroître la sécurité de l'hydrogel injecté en améliorant sa capacité à s'intégrer / s'implanter dans les tissus
- d'accroître considérablement la performance de l'hydrogel injecté en améliorant notamment sa capacité à créer davantage de volume au cours du temps

Ainsi, la présente invention concerne, selon un premier de ses aspects, une nouvelle composition aqueuse stérile injectable, stérilisée à la chaleur, contenant au moins de l'acide hyaluronique réticulé, ou l'un de ses sels, et un ou plusieurs acides gras; composition sous forme de gel contenant plus de 51% en masse d'eau et moins de 45% en masse d'acide gras; composition possédant des propriétés de viscoélasticité telles que le rapport G"/G' à 0.7 Hz est inférieur à 0.70.

Selon l'invention, la composition contient de l'acide hyaluronique ou l'un de ses sels, et en particulier ses sels acceptables d'un point de vue physiologique comme les sels de sodium, calcium, zinc, potassium, avantageusement le sel de sodium. L'acide hyaluronique peut être d'origine animale ou obtenu par fermentation bactérienne. Il peut avoir une masse moléculaire de quelques daltons à plusieurs millions de daltons, avantageusement d'environ 0.01 à 5 millions de daltons, encore avantageusement d'environ 0.1 à 3.5 millions de daltons.

Selon un aspect de l'invention, la composition peut être à base d'un dérivé de l'acide hyaluronique, c'est-à-dire à base d'une molécule obtenue en modifiant par voie chimique, ou par toute autre voie, la molécule d'acide hyaluronique.

Selon l'invention, la concentration totale en acide hyaluronique, ou l'un de ses sels, est comprise entre 0.001 et 70 mg/ml, entre 0.01 et 50 mg/ml, entre 1 et 40 mg/ml, entre 5 et 35 mg/ml, entre 8 et 33 mg/ml, entre 9 et 30 mg/ml, entre 10 et 29 mg/ml, entre 11 et 28 mg/ml, entre 12 et 27 mg/ml, entre 13 et 26.5 mg/ml, avantageusement entre 14 et 26 mg/ml.

Selon l'invention, l'acide hyaluronique contenu dans la composition est réticulé, préférentiellement selon les techniques de réticulation décrites dans l'art antérieur. Le ou les agents réticulants qui interviennent dans la réticulation peuvent être identiques ou différents. Ce sont généralement des réticulants bi- ou poly-fonctionnels de différents types et ils peuvent par exemple être sélectionnés parmi la divinylsulfone, les époxy bi- ou poly-fonctionnels, les carbodiimides et le formaldéhyde. On choisit de préférence les agents de la famille des époxy bi- ou poly-fonctionnels et notamment le 1,4-butanedioldiglycidyléther (BDDE), le diépoxy-octane ou le 1,2-bis-(2,3-époxypropyl)-2,3-éthylène. On préfère tout particulièrement utiliser le BDDE. Les températures de réticulation sont généralement comprises entre environ 15°C et 60°C et les durées de réticulation sont généralement de plusieurs heures, avantageusement de plus de 1h jusqu'à environ 24h.

Selon l'invention, la composition contient une proportion massique en acide gras inférieure à 45% de la masse totale, inférieure à 40% de la masse totale, inférieure à 35% de la masse totale, inférieure à 30% de la masse totale, inférieure à 25% de la masse totale, inférieure à 20% de la masse totale, inférieure à 18% de la masse totale, inférieure à 17% de la masse totale, inférieure à 16% de la masse totale, inférieure à 15% de la masse totale, inférieure à 14% de la masse totale, inférieure à 13% de la masse totale, inférieure à 12% de la masse totale, inférieure à 11% de la masse totale, inférieure à 10% de la masse totale, inférieure à 9% de la masse totale, inférieure à 8% de la masse totale, inférieure à 7% de la masse totale.

Selon l'invention, la composition contient un ou plusieurs acides gras. On peut citer par exemple les acides gras suivants :
- les acides gras saturés : acide butanoïque, acide pentanoïque, acide hexanoïque, acide heptanoïque, acide octanoïque, acide nonanoïque, acide décanoïque, acide undécanoïque, acide dodécanoïque, acide tridécanoïque, acide tétradecanoïque, acide pentadécanoïque, acide hexadécanoïque, acide heptadécanoïque, acide octadécanoïque, acide nonadécanoïque, acide eicosanoïque, acide heneicosanoïque, acide docosanoïque, acide tricosanoïque, acide tétracosanoïque, acide pentacosanoïque, acide hexacosanoïque, acide heptacosanoïque, acide octacosanoïque, acide nonacosanoïque, acide triacontanoïque, acide tétracontanoïque, acide pentacontanoïque, acide hexacontanoïque, acide heptacontanoïque, acide octacontanoïque, acide nonacontanoïque, acide hectanoïque, acide dictanoïque. On choisit avantageusement l'acide butanoïque (couramment appelé acide butyrique), l'acide hexanoïque (couramment appelé acide caproïque), l'acide octanoïque (couramment appelé acide caprylique), l'acide décanoïque (couramment appelé acide caprique), l'acide dodécanoïque (couramment appelé acide laurique), l'acide tétradecanoïque (couramment appelé acide myristique), l'acide hexadécanoïque (couramment appelé acide palmitique) et l'acide octadécanoïque (couramment appelé acide stéarique).

- les acides gras mono-insaturés : acide dodécénoïque, acide tétradecénoïque, acide hexadécénoïque, acide octadécénoïque, acide icosénoïque, acide docosénoïque, acide tétracosénoïque. On choisit avantageusement l'acide hexadécénoïque (couramment appelé acide palmitoléique) et l'acide octadécénoïque (couramment appelé acide oléique).
- les acides gras poly-insaturés : acide octadécadiénoïque, acide octadécatriénoïque, acide octadécatétraénoïque, acide icosatétraénoïque, acide icosapentaénoïque, acide docosapentaénoïque, acide docosahexaénoïque. On choisit avantageusement l'acide octadécadiénoïque (couramment appelé acide linoléique), l'acide octadécatriénoïque (couramment appelé acide alpha-linoléique), et l'acide icosatétraénoïque (couramment appelé acide arachidonique).

Selon un aspect de l'invention, la concentration totale en acide gras est supérieure à 0.0001 mg/ml, supérieure à 0.001 mg/ml, supérieure à 0.01 mg/ml, supérieure à 0.1 mg/ml, supérieure à 1 mg/ml, supérieure à 10 mg/ml, supérieure à 20 mg/ml, supérieure à 30 mg/ml, supérieure à 40 mg/ml, supérieure à 50 mg/ml, supérieure à 60 mg/ml, supérieure à 70 mg/ml, supérieure à 80 mg/ml, supérieure à 90 mg/ml, supérieure à 100 mg/ml, supérieure à 110 mg/ml, supérieure à 120 mg/ml, supérieure à 130 mg/ml.

Selon l'invention, la composition a pour ingrédient majoritaire l'eau (en proportion massique), d'où le qualificatif de composition aqueuse pour la nouvelle composition selon l'invention. La masse en eau dans la composition selon l'invention est supérieure à 51% de la masse totale, avantageusement supérieure à 60% de la masse totale, avantageusement supérieure à 70% de la masse totale, avantageusement supérieure à 75% de la masse totale, avantageusement supérieure à 80% de la masse totale, avantageusement supérieure à 85% de la masse totale. Une solution tampon est avantageusement utilisée notamment pour mieux contrôler le pH et l'osmolarité de la formulation tout au long de sa durée de péremption. On peut par exemple citer l'utilisation d'un tampon à base de chlorure de sodium et d'ions phosphates.

Selon l'invention, la composition est stérile. Elle est stérilisée selon les techniques décrites dans l'art antérieur. Elle est avantageusement stérilisée à la chaleur, préférentiellement à la chaleur humide (également appelée autoclavage à la vapeur).

De manière préférée, la stérilisation à la chaleur humide est effectuée à une température supérieure à 100°C, avantageusement supérieure à 110°C, avantageusement supérieure à 120°C. De manière générale, la durée de stérilisation peut aller de quelques secondes à plusieurs minutes. On peut citer par exemple les cycles de stérilisation à la chaleur humide suivants : 121°C pendant 20 minutes ou 125°C pendant 7 minutes ou 127°C pendant 4 minutes ou encore 130°C pendant 3 minutes.

Il est important de noter que la stérilisation à la chaleur est avantageusement sélectionnée car elle octroie notamment un très haut niveau de stérilité à la composition choisie, ce qui permet alors de viser un haut niveau de sécurité pour le patient traité.

Selon l'invention, la composition est injectable. Elle est préférentiellement conditionnée dans une seringue ou dans un vial, afin de pouvoir être facilement administrée à travers une aiguille ou une canule.

Selon l'invention, la composition possède des propriétés rhéologiques de viscoélasticité telles que le rapport G"/G' (= Tan δ) à la fréquence de 0.7 Hz est inférieur à 0.70, préférentiellement inférieur à 0.65, encore préférentiellement inférieur à 0.60, encore préférentiellement inférieur à 0.55, encore préférentiellement inférieur à 0.50, encore préférentiellement inférieur à 0.45, encore préférentiellement inférieur à 0.40, encore préférentiellement inférieur à 0.35.

Les modules G' (= module élastique) et G" (= module visqueux), bien connus par l'homme de l'art pour la caractérisation des hydrogels à base d'acide hyaluronique réticulé (par exemple, Sundaram et al., Plast Reconst Surg, 2013, 132:5S-21S), sont mesurés à l'aide d'un rhéomètre, par exemple un rhéomètre à contrainte imposée, à la température de 25°C, avec une géométrie de 4 cm, en effectuant un balayage en fréquence avec une déformation contenue dans le domaine de viscoélasticité linéaire.

Selon l'invention, les molécules d'acide gras sont dispersées dans l'hydrogel de manière homogène ou relativement homogène (dispersion de fines gouttelettes d'acide gras dans l'hydrogel), sans que cela forme une composition biphasique, c'est-à-dire une composition dans laquelle la phase aqueuse et la phase organique sont complètement séparées l'une de l'autre (par exemple, lorsque l'on observe des zones importantes de coalescence de la phase organique au sein de l'hydrogel ou que la phase organique se retrouve complètement au-dessus de la phase aqueuse de l'hydrogel).

Selon un aspect préféré de l'invention, la composition selon l'invention ne contient pas de tensioactifs ayant pour objectif de stabiliser le mélange d'acide gras dans l'eau ; ces molécules additionnelles étant susceptibles d'altérer le haut niveau de sécurité impérativement requis pour ce type de formulations injectables.

Selon un aspect de l'invention, la composition comprend également des liposomes ou des niosomes contenant un ou plusieurs acides gras et/ou toute autre substance d'intérêt pour la formulation envisagée. On entend par substance d'intérêt toute molécule susceptible d'apporter un bénéfice à la composition et/ou à l'organisme dans laquelle la composition est administrée.

Les liposomes et les niosomes sont de petites vésicules sphériques dont la paroi est formée d'une ou plusieurs couches lipidiques, renfermant un espace interne aqueux. Leur intérêt réside principalement dans la possibilité de vectoriser des substances, soit par inclusion dans la membrane lipidique, soit par encapsulation dans la membrane interne. Il est par exemple possible d'y encapsuler des principes actifs de solubilité très différentes (hydrophile, amphiphile ou lipophile).

Les liposomes sont obtenus à partir de molécules amphiphiles naturelles (phospholipides ou stéroïdes), et les niosomes sont composés de molécules amphiphiles synthétiques non-ioniques (lipopolyglycérols). Leurs structures sont microscopiques (0.01 micron à plusieurs microns).

Selon un aspect de l'invention, la composition selon l'invention contient une ou plusieurs substances actives d'origine naturelle ou synthétique à action pharmacologique ou non, comme par exemple les antioxydants, les anti-inflammatoires, les antiseptiques, les antibactériens, les antifongiques, les anticancéreux, les anesthésiques locaux, les protéines, les hormones, seuls ou en combinaison. Ces substances actives sont soit dispersées dans l'hydrogel, soit greffées à un ou plusieurs des polymères de l'hydrogel, soit contenues/encapsulées dans des liposomes/niosomes dispersés dans l'hydrogel, soit contenues/encapsulées dans un autre matériau lui-même dispersé au sein de l'hydrogel.

Selon un aspect de l'invention, la famille des acides gras est élargie aux lipides biologiquement acceptables, c'est-à-dire aux lipides possédant un haut niveau de biocompatibilité dans l'organisme.

Ainsi, selon un aspect de l'invention, la nouvelle composition aqueuse stérile injectable, stérilisée à la chaleur, comprend au moins de l'acide hyaluronique réticulé, ou l'un de ses sels, et un ou plusieurs lipides biologiquement acceptables, caractérisée en ce que :
- la proportion massique en eau est supérieure à 51% de la masse totale
- la proportion massique en lipide est inférieure à 45% de la masse totale
- les propriétés de viscoélasticité sont telles que le rapport G"/G' à 0.7 Hz est inférieur à 0.70

Selon un aspect avantageux de l'invention, la composition selon l'invention contient de la lidocaïne dispersée au sein de l'hydrogel.

Selon un aspect de l'invention, la composition selon l'invention contient un ou plusieurs composés d'origine biologique comme des cellules, des plaquettes enrichies, des gènes, des fragments d'ADN ou des facteurs de croissance. Ces composés sont préférentiellement dispersés dans l'hydrogel, mais ils peuvent également être greffés à un ou plusieurs des polymères de l'hydrogel ou contenus/encapsulés dans des liposomes/niosomes dispersés dans l'hydrogel ou contenus/encapsulés dans un autre matériau lui-même dispersé au sein de l'hydrogel.

Selon un aspect de l'invention, la composition selon l'invention contient des polymères qui sont dispersés au sein de la matrice réticulée de l'hydrogel. On peut citer par exemple les polymères de la famille des polysaccharides, les polyesters, les polyanhydrides, les polyphosphazenes, les poly-ε-caprolactones, les acides polylactiques et leurs dérivés, les acides polyvinyliques, les polyacrylamides, la N-vinyl pyrrolidone et les polymères acryliques et dérivés biologiquement acceptables.

Selon un aspect de l'invention, la composition selon l'invention contient des substances minérales qui sont dispersées au sein de la matrice réticulée de l'hydrogel. On peut citer par exemple l'hydroxyapatite ou les phosphates tricalciques comme le β tricalcium phosphate.

Selon un aspect de l'invention, la composition selon l'invention est mélangée avec une ou plusieurs autres substances, préférentiellement stériles, susceptibles d'apporter un bénéfice à l'organisme, juste avant son administration chez le patient. Le mélange est alors effectué par l'utilisateur final, c'est-à-dire par un praticien ou par du personnel habilité, selon une méthode appropriée utilisant un ou plusieurs dispositifs de mélange permettant de réaliser un mélange satisfaisant et de conserver la stérilité. On peut par exemple citer le mélange par l'utilisateur final de l'hydrogel selon l'invention et d'un ou plusieurs composés comme des acides gras, des lipides, des substances actives, des biologiques ou des substances minérales :
- en réalisant des allers-retours entre deux contenants (l'un rempli de l'hydrogel selon l'invention et l'autre rempli du composé à disperser dans l'hydrogel), ces contenants pouvant être par exemple des seringues
- en extrudant simultanément le contenu de deux contenants (l'un rempli de l'hydrogel selon l'invention et l'autre rempli du composé à disperser dans l'hydrogel) pour réunir les différents composés et/ou les mélanger dans un autre contenant avant administration chez le patient

La nouvelle composition divulguée dans la présente invention a pour objectif d'améliorer la sécurité et la performance/efficacité clinique des produits injectables à base d'acide hyaluronique réticulé en procurant notamment les bénéfices suivants :
- meilleure intégration de la composition selon l'invention sur son site d'implantation, au niveau de la zone traitée. En effet, par rapport à une formulation à base d'acide hyaluronique réticulé de l'art antérieur, la composition aqueuse selon l'invention contient des acides gras (phase organique dispersée dans l'hydrogel), ce qui lui confère une plus grande proximité physiologique avec les tissus environnants, en particulier lorsque le produit est injecté dans des zones à forte densité graisseuse (cas par exemple des injections sous-cutanées dans le visage et notamment des injections pour augmenter le volume des joues ou du menton). Ainsi, la composition selon l'invention est avantageusement injectée dans les tissus sous-cutanés et/ou dans tout tissu contenant partiellement ou totalement de la graisse.
   Les injections de gels à base d'acide hyaluronique réticulé sont connues pour posséder un haut niveau de sécurité. La solution selon l'invention, de par sa capacité à mieux s'intégrer dans les tissus environnants, a néanmoins la capacité d'améliorer significativement ce niveau de sécurité en réduisant les effets secondaires en post injection : moins d'inflammation du fait d'une reconnaissance plus favorable de l'implant par l'organisme d'où par exemple moins de rougeurs et/ou de douleurs suite à l'injection
- meilleur environnement offert par la composition selon l'invention aux cellules qui se retrouvent alors à proximité et/ou en contact avec des acides gras; acides gras susceptibles d'être stockés au sein des cellules adipocytes, induisant ainsi une augmentation du volume cellulaire, et/ou d'être utilisés comme matières premières/nourriture par les cellules, ce qui peut participer à les stimuler/renforcer
- meilleure durabilité/rémanence du traitement grâce à un effet synergique permettant de combiner la capacité de création de volume par l'acide hyaluronique réticulé et la capacité de stimulation des cellules par les acides gras. Il est à noter que cette synergie peut potentiellement être accrue/optimisée si des acides gras sont encapsulés (par exemple dans des liposomes ou dans des niosomes), partiellement ou totalement, pour permettre une libération dans la durée de ceux-ci
- meilleure capacité à disperser des molécules organiques dans la composition selon l'invention (par rapport à un hydrogel à base d'acide hyaluronique réticulé sans acide gras) du fait de la présence d'une fraction organique représentée par les acides gras; ce qui peut permettre par exemple d'introduire davantage de molécules de nature lipophile dans le produit et/ou de viser une meilleure homogénéité de celui-ci
- la possibilité de limiter le phénomène indésirable de création d'œdème (effet secondaire bien connu dans le cas des traitements à base d'acide hyaluronique réticulé) en post injection du fait d'une réduction de l'inflammation au niveau de la zone injectée (car meilleure intégration du produit dans les tissus) et/ou d'une plus faible capacité du produit à gonfler *in vivo* dans les tissus (la présence d'une fraction organique pouvant limiter la capacité de l'acide hyaluronique réticulé à capter de l'eau *in vivo* impliquant ainsi un plus faible gonflement du gel au niveau de la zone traitée)

Il est également important de noter que, selon un aspect de l'invention, les acides gras peuvent être encapsulés dans des liposomes/niosomes en étant distribués soit dans la bicouche lipidique, soit à l'intérieur de la phase aqueuse.

Dans ce cas de l'encapsulation d'un ou plusieurs acides gras et/ou de toute autre substance d'intérêt dans des liposomes/niosomes, les bénéfices suivants peuvent être mis en avant :
- le maintien de l'intégrité d'une substance d'intérêt au sein d'un gel d'acide hyaluronique réticulé : effectivement, grâce à l'intégration de la substance d'intérêt dans ces liposomes/niosomes, la substance d'intérêt n'est pas dégradée/oxydée, et/ou ne réagit pas avec la matrice d'acide hyaluronique réticulé, que ce soit lors du procédé de fabrication de la composition selon l'invention, ou lors du contact avec les cellules cibles après que la composition ait été injectée et que les liposomes/niosomes aient été libérés de l'hydrogel
- la libération prolongée dans le temps : le liposome/niosome (encapsulant la substance d'intérêt), de taille nanométrique/micrométrique, va se libérer lentement de l'hydrogel du fait de l'encombrement stérique au sein du réseau tridimensionnel d'acide hyaluronique réticulé : Ainsi, de manière générale, la substance d'intérêt encapsulée est libérée sur des temps plus longs par rapport à une simple dispersion de cette substance dans la matrice d'acide hyaluronique réticulé : l'effet biologique recherché (par exemple, stimulation des cellules type fibroblastes et/ou adipocytes) est ainsi plus marqué dans le ' temps
- le ciblage de la substance d'intérêt : une fois libérée de la matrice d'acide hyaluronique réticulé, le liposome/niosome va être éliminé (par phagocytose) et cette endocytose va augmenter la concentration en substance d'intérêt (par exemple en acide gras) au niveau cellulaire (lysosomes) rendant ainsi l'action de la substance d'intérêt plus efficace sur les cellules cibles
- la solubilisation de substances d'intérêt (par exemple des acides gras) peu solubles dans l'eau; le liposome/niosome permettant ainsi de garder un produit avec une seule phase aqueuse. En effet, les liposomes/niosomes, du fait de leur hydrophilie en surface de vésicule, sont en mesure de favoriser l'intégration et l'homogénéisation de la substance d'intérêt dans la matrice tridimensionnelle d'acide hyaluronique réticulé

De manière surprenante, il est important de noter que la composition selon l'invention, de part ses caractéristiques spécifiques et notamment ses propriétés rhéologiques/mécaniques particulières :
- possède un aspect macroscopique homogène, bien que constitué d'eau (ingrédient majoritaire) et d'acide gras, et ceci, même en l'absence d'émulsifiant.
   Les acides gras (phase organique) sont dispersés dans l'hydrogel (phase aqueuse), formant ainsi un gel d'aspect macroscopique homogène, tout en étant microscopiquement hétérogène. Sans vouloir se lier à une explication, on peut supposer que l'acide hyaluronique réticulé, dans les conditions de l'invention (notamment G"/G' < 0.70), joue le rôle de stabilisateur d'une émulsion huile dans eau
- forme une émulsion stable même dans le cas d'une stérilisation à la chaleur, et ceci même pour des cycles de stérilisation durant plusieurs dizaines de minutes à plus de 120°C
- forme une émulsion stable au cours du temps sur une durée de plusieurs mois à température ambiante
- possède une cinétique de libération des acides gras hors de l'hydrogel extrêmement lente; libération pouvant être qualifiée de retardée. En effet, alors qu'il est bien connu par l'homme de l'art que des petites molécules d'intérêt, voire des molécules de plus grande taille de plus de 5000 g/mol, dispersées dans un gel à base d'acide hyaluronique réticulé, ont des cinétiques de libération de quelques heures (cas de la lidocaïne ou des polyols comme le mannitol ou le glycérol), limitant ainsi fortement leur efficacité clinique sur le moyen ou long terme; les acides gras dispersés dans le gel selon l'invention sont libérés sur des temps particulièrement longs. Cette caractéristique (libération retardée) confère un avantage considérable à l'hydrogel selon l'invention et elle est obtenue sans même faire appel à des techniques contraignantes et coûteuses de greffage et/ou de microencapsulation. Sans vouloir se lier à une explication, on peut supposer que la stérilisation à la chaleur joue un rôle bénéfique pour la composition selon l'invention en créant une affinité particulière entre l'acide hyaluronique réticulé et les acides gras; affinité favorisant une libération retardée sur le long terme des acides gras hors de l'hydrogel.

Ces éléments surprenants sont illustrés dans les exemples.

De manière surprenante, il est également important de noter que la composition selon l'invention possède des propriétés rhéologiques/mécaniques (cas des modules viscoélastiques G' et G") et de cohésivité similaires à celles d'une composition à base d'acide hyaluronique réticulé ne contenant pas d'acide gras. Ces propriétés sont décrites comme étant clés dans la littérature pour la sécurité et la performance clinique d'un produit injectable à base d'acide hyaluronique réticulé (par exemple, Sundaram et al., Plast Reconst Surg, 2013, 132:5S-21S). En d'autres termes, l'ajout d'acide gras, dans les conditions de l'invention, même en quantité relativement importante, impacte peu les propriétés rhéologiques/mécaniques et la cohésivité de l'hydrogel. Ce point est particulièrement intéressant car il offre la possibilité de fournir aux praticiens des produits injectables avec acides gras possédant des propriétés similaires à celles déjà connues actuellement, et donc ayant vocation à traiter les mêmes indications, tout en visant une meilleure sécurité et performance clinique (par exemple, en offrant une efficacité clinique, comme la création de volume au niveau des tissus, améliorée sur le long terme).

Cet élément surprenant est également illustré dans les exemples.

Il est également important de noter que la composition selon l'invention possède une résistance à la dégradation enzymatique et radicalaire (facteurs clés de la dégradation *in vivo* d'un hydrogel à base d'acide hyaluronique) similaire à celle de la composition de l'art antérieur ne contenant pas d'acide gras. De même que précédemment, cette caractéristique est avantageuse car elle permet de proposer aux praticiens des produits pouvant être administrés pour les mêmes indications que celles traitées à ce jour, tout en apportant des bénéfices supplémentaires procurés par la combinaison entre l'acide hyaluronique réticulé et les acide gras dans les conditions de l'invention.

Cet élément surprenant est également illustré dans les exemples.

De manière surprenante, il est également important de citer que la composition selon l'invention possède une force d'éjection (= force permettant d'expulser le gel conditionné en seringue sur laquelle est montée une aiguille ou une canule) similaire à celle d'une composition à base d'acide hyaluronique réticulé ne contenant pas d'acide gras. En d'autres termes, l'ajout d'acide gras, dans les conditions de l'invention, même en quantité relativement importante, impacte peu les propriétés d'extrusion de l'hydrogel. Cette observation est particulièrement surprenante car l'homme de l'art s'attendrait à obtenir des forces d'éjection plus faibles pour la composition selon l'invention par rapport à des compositions à base d'acide hyaluronique réticulé ne contenant pas d'acide gras, connaissant le pouvoir lubrifiant des acides gras. Ce point est également intéressant, car il permet de conserver des forces d'éjection appropriées et habituellement connues pour le praticien injecteur, évitant ainsi d'injecter des quantités trop importantes (si la force d'éjection était plus faible car moins de résistance à la poussée/extrusion), limitant ainsi les effets indésirables relatifs au surdosage du produit administré.

Cet élément surprenant est également illustré dans les exemples.

D'autre part, il est important de mettre en évidence les effets synergiques suivants relatifs à la sécurité et à la performance clinique de la composition selon l'invention :
- dans les conditions de l'invention, l'acide hyaluronique réticulé permet de retenir les acides gras au niveau de la zone traitée pour une efficacité sur le plus long terme (alors qu'il y a diffusion immédiate des acides gras si l'injection est effectuée en solution, sans acide hyaluronique) alors que les acides gras, eux, améliorent la capacité de bio-intégration de l'acide hyaluronique réticulé qui est élastique/rigide et de nature hydrophile, dans un environnement partiellement ou totalement graisseux
- dans les conditions de l'invention, l'acide hyaluronique réticulé, de par son élasticité/rigidité (faible ratio G"/G', synonyme d'un fort caractère élastique de la composition), est en mesure de créer un stress mécanique par compression sur les cellules de la zone traitée, et notamment sur les cellules fibroblastes et/ou adipocytes. Comme décrit dans la littérature, un stress mécanique donne la possibilité de stimuler les cellules leur permettant alors notamment de relancer et/ou produire davantage de composés bénéfiques à l'organisme comme par exemple en produisant davantage les constituants clés de la matrice extra-cellulaire (collagène, élastine, acide hyaluronique, ...), ce qui permet alors d'améliorer et/ou de prolonger l'effet long terme du produit injecté. D'autre part, la présence d'acides gras, poussés à la surface des cellules permet encore d'améliorer le phénomène décrit précédemment en favorisant la stimulation des cellules, notamment en servant de matières premières/nourriture et/ou de catalyseur pour la production de composés bénéfiques par les cellules ou encore en étant tout simplement stockés au sein des cellules adipocytes (ce qui génère alors une augmentation du volume cellulaire allant dans le sens de l'effet clinique recherché, c'est-à-dire une augmentation du volume des tissus traités).

La présente invention concerne, selon un deuxième de ses aspects, un procédé de préparation de la nouvelle composition stérile injectable décrite précédemment.

Le procédé de préparation de la composition selon l'invention se caractérise par les étapes successives suivantes :
a) préparation d'un hydrogel à base d'acide hyaluronique réticulé, ou l'un de ses sels
b) ajout du ou des acides gras dans l'hydrogel avec opération de mélange
c) stérilisation

L'étape (a) débute généralement par la mise en solution de l'acide hyaluronique puis par sa réticulation, à l'aide d'un réticulant, et elle se termine généralement par la purification de l'hydrogel obtenu.

On entend par étape de réticulation de l'acide hyaluronique, l'étape permettant de ponter les chaînes d'acide hyaluronique les unes aux autres par des liaisons covalentes. De manière générale, l'étape de réticulation de l'acide hyaluronique commence lorsque le réticulant est mis en contact avec l'acide hyaluronique et se termine lorsque l'homme de l'art considère que la cinétique de réaction de pontage des chaînes d'acide hyaluronique par le réticulant a atteint un niveau négligeable.

La purification de l'hydrogel, elle, permet de retirer les molécules indésirables du gel préparé et en particulier les résidus de réticulant, suite à la réticulation. Cette purification est effectuée selon les techniques bien connues par l'homme de l'art comme par exemple par bains de dialyse, par lavage par flux d'eau continu ou par précipitation.

La préparation d'un hydrogel à base d'acide hyaluronique réticulé est effectuée avantageusement selon les méthodes décrites dans l'art antérieur. On pourra par exemple citer les demandes WO 97/04012, WO 2004/092222, WO 2005/085329 et WO 2009/071697 pour la fabrication d'un hydrogel à base d'acide hyaluronique réticulé.

L'étape (b) consiste à ajouter un ou plusieurs acides gras dans l'hydrogel à base d'acide hyaluronique réticulé préalablement préparé.

Dans le but de favoriser l'homogénéisation des acides gras dans l'hydrogel à base d'acide hyaluronique réticulé, il est à noter que ces acides gras peuvent être chauffés avant leur ajout dans le gel, ceci en particulier lorsque ces acides gras se présentent sous la forme solide à température ambiante, permettant ainsi leur transformation en un liquide pouvant être dispersé dans l'hydrogel.

Cet ajout peut être effectué par exemple :
- soit en ajoutant directement dans l'hydrogel le ou les acides gras (à l'état pur)
- soit en encapsulant le ou les acides gras par exemple dans des liposomes/niosomes
- soit en préparant au préalable une solution contenant le ou les acides gras (encapsulés ou non)
- soit en ajoutant un composé additionnel sur lequel le ou les acides gras ont été greffés

Dans le cas de l'encapsulation d'acide gras dans des liposomes/niosomes, ces derniers sont préparés préalablement à leur ajout dans le gel réticulé d'acide hyaluronique.

Plusieurs méthodes de préparation des liposomes/niosomes selon l'invention sont envisageables :
- méthodes classiques faisant intervenir une étape mécanique de dispersion des phospholipides, comme la sonication, l'extrusion ou la microfluidisation
- méthodes basées sur l'hydratation d'un film lipidique (les lipides et les molécules d'intérêt à encapsuler sont tout d'abord solubilisés dans un solvant organique puis le solvant est évaporé. Le film obtenu est ensuite hydraté dans une solution aqueuse)
- méthodes basées sur l'élimination du solvant organique à partir d'une émulsion (évaporation en phase inverse)
- méthodes reposant sur l'élimination d'un détergent à partir d'une solution de micelles mixtes
- méthodes basées sur la transformation de liposomes pré-formés, comme la lyophilisation/réhydratation ou la congélation/décongélation

Cet ajout du ou des acides gras s'accompagne d'une opération de mélange ayant vocation à homogénéiser le ou les acides gras au sein de l'hydrogel. Le mélange peut se faire pendant l'ajout et/ou après l'ajout.

L'opération de mélange est effectuée par les techniques bien connues par l'homme de l'art comme par exemple par mélange mécanique au sein d'une cuve de mélange.

Le mélange peut être effectué à une température supérieure à la température ambiante et/ou sous pression et/ou sous ultra-sons afin d'optimiser l'homogénéisation de la composition.

L'étape (c) consiste à stériliser la composition par les techniques bien connues par l'homme de l'art; la stérilisation étant avantageusement réalisée à la chaleur par autoclavage.

Un procédé avantageux selon l'invention pour la fabrication d'une composition aqueuse stérile injectable selon l'invention comprend au moins les étapes suivantes :
- préparation d'une solution aqueuse d'acide hyaluronique
- réticulation de l'acide hyaluronique
- purification de l'hydrogel, par exemple par dialyse dans une solution physiologique
- ajout d'un ou plusieurs acides gras avec opération de mélange
- conditionnement en seringues
- stérilisation

Un autre procédé avantageux selon l'invention pour la fabrication d'une composition aqueuse stérile injectable selon l'invention comprend au moins les étapes suivantes :
- préparation d'une solution aqueuse d'acide hyaluronique
- réticulation de l'acide hyaluronique
- purification de l'hydrogel, par exemple par dialyse dans une solution physiologique
- incorporation d'un ou plusieurs acides gras dans des liposomes/niosomes et ajout de ces liposomes/niosomes dans l'hydrogel avec opération de mélange
- conditionnement en seringues
- stérilisation

La présente invention concerne, selon un troisième de ses aspects, l'utilisation chez l'homme ou chez l'animal de la nouvelle composition aqueuse stérile injectable décrite précédemment, pour des applications esthétiques ou thérapeutiques.

La composition aqueuse stérile injectable selon l'invention est notamment utilisée pour :
- combler des volumes
- générer des espaces au sein de certains tissus, favorisant ainsi leur fonctionnement optimal
- remplacer des liquides physiologiques ou des tissus déficients
- stimuler ou favoriser la régénération des tissus
- hydrater et protéger des tissus
- délivrer des substances susceptibles d'apporter un bénéfice à l'organisme et notamment des substances actives et/ou des biologiques

A titre d'exemple, on peut citer les utilisations de l'hydrogel dans les cas suivants :
- la formulation d'une composition injectable en intradermique ou en sous-cutané pour l'amélioration de la qualité de la peau ou le comblement des rides ou la restauration des volumes du visage (pommettes, menton, lèvres, nez, ...) ou du corps
- la formulation d'une composition injectable à usage dentaire par exemple pour combler des poches parodontales et/ou pour stimuler la régénération des tissus autour de la dent
- la formulation d'une composition injectable en intra-oculaire, notamment pour des applications au cours de la chirurgie de la cataracte, du glaucome, de la presbytie ou du vitré
- la formulation d'une composition injectable en intra-articulaire pour des applications en orthopédie ou en rhumatologie, notamment dans le cadre de la viscosupplémentation du liquide synovial déficient pour le traitement de l'arthrose mais également de la reconstruction osseuse ou de la régénération du cartilage
- la formulation d'une composition injectable en urologie pour des applications dans le traitement de l'incontinence urinaire ou fécale
- la formulation d'une composition injectable utilisée en médecine ou chirurgie générale dans le cadre du traitement de la fibrose ou pour améliorer la cicatrisation des plaies
- la formulation d'une composition pharmaceutique injectable permettant la libération contrôlée de substances actives et/ou de biologiques pour différentes applications médicales

### EXEMPLES

L'invention va maintenant être illustrée, de manière non limitative, par les exemples suivants.

Le hyaluronate de sodium, les acides gras et l'ensemble des autres composés utilisés dans les exemples suivants possèdent un haut niveau de pureté.

L'extrusion des gels est déterminée par la mesure de la force nécessaire (en Newton) pour éjecter la composition contenue dans une seringue type, à travers une aiguille de 27G ½ à une vitesse de 13.5 mm/min.

Les propriétés rhéologiques (mesure du module élastique G' et de Tanδ = G"/G') des gels sont mesurées à 25°C à l'aide d'un rhéomètre à contrainte imposée (TA AR2000) et d'une géométrie cône/plan 4 cm-2° avec un entrefer de 1000 micromètres.

### Exemple 1 : Préparation d'un gel G1 selon l'invention et du gel REF1 (comparatif)

On pèse 1.23 g d'une poudre de hyaluronate de sodium (NaHA), de masse moléculaire environ égale à 1.5 MDa, avec un taux d'humidité de 8.2%, auxquels on ajoute 11.3 g d'une solution aqueuse de NaOH à 0.25 N. L'hydratation de la poudre dure 1h30, avec une homogénéisation manuelle régulière à la spatule. 0.63 g d'une solution de 1,4-butanedioldiglycidyléther (BDDE) dilué au 1/5^{ème} dans la soude 0.25 N sont ajoutés au milieu réactionnel, suivi d'une homogénéisation mécanique de 15 minutes avant immersion dans un bain thermostaté à 50°C pendant 2h30. On ajoute une solution de tampon phosphate contenant du HCl dans le réticulat obtenu afin d'obtenir un pH=7.3 et une concentration en acide hyaluronique égale à 25 mg/ml. On laisse le gel gonfler pendant 24h à température ambiante dans cette solution et, à la fin de ce temps, on l'homogénéise manuellement à la spatule pendant 10 minutes avant de le purifier par dialyse pendant 24h en utilisant une membrane à base de cellulose (seuil de rétention = 10 000 Da) dans une solution de tampon phosphate. On mélange manuellement le gel à la spatule pendant 10 minutes. Soit REF1A le gel ainsi obtenu. La concentration en acide hyaluronique de REF1A est de 19.9 mg/ml.

Dans 15.0 g de gel REF1A, on ajoute 2.7 g d'acide palmitoléique. On mélange manuellement le gel à la spatule pendant 10 minutes. Soit GEL1A le gel ainsi obtenu.

Les gels REF1A et GEL1A sont conditionnés en seringues de 1 ml puis stérilisés à l'autoclave à 121°C pendant 10 minutes. Soit REF1 et GEL1 (= gel selon l'invention), les gels stérilisés issus de REF1A et GEL1A.

### Exemple 2 : Préparation d'un gel G21 selon l'invention et du gel REF21 (comparatif)

On pèse 8.31 g d'une poudre de hyaluronate de sodium (NaHA), de masse moléculaire environ égale à 1.7 MDa, avec un taux d'humidité de 4.9%, auxquels on ajoute 73.5 g d'une solution aqueuse de NaOH à 0.25 N. L'hydratation de la poudre dure 1h30, avec une homogénéisation manuelle régulière à la spatule. 4.37 g d'une solution de 1,4-butanedioldiglycidyléther (BDDE) dilué au 1/5^{ème} dans la soude 0.25 N sont ajoutés au milieu réactionnel, suivi d'une homogénéisation mécanique de 15 minutes avant immersion dans un bain thermostaté à 50°C pendant 2h30. On ajoute une solution de tampon phosphate contenant du HCl dans le réticulat obtenu afin d'obtenir un pH=7.3 et une concentration en acide hyaluronique égale à 30 mg/ml. On laisse le gel gonfler pendant 24h à température ambiante dans cette solution et, à la fin de ce temps, on l'homogénéise manuellement à la spatule pendant 10 minutes avant de le purifier par dialyse pendant 24h en utilisant une membrane à base de cellulose (seuil de rétention = 10 000 Da) dans une solution de tampon phosphate. On mélange manuellement le gel à la spatule pendant 10 minutes. Soit REF2A le gel ainsi obtenu. La concentration en acide hyaluronique de REF2A est de 25.2 mg/ml.

Dans 45.0 g de gel REF2A, on ajoute 2.3 g d'acide oléique. On mélange manuellement le gel à la spatule pendant 10 minutes. Soit GEL2A1 le gel ainsi obtenu.

Dans 45.0 g de gel REF2A, on ajoute 2.3 g d'une solution tampon phosphate. On mélange manuellement le gel à la spatule pendant 10 minutes. Soit REF2A1 le gel ainsi obtenu.

Les gels REF2A1 et GEL2A1 sont conditionnés en seringues de 1 ml puis stérilisés à l'autoclave à 121°C pendant 10 minutes. Soit REF21 et GEL21 (= gel selon l'invention), les gels stérilisés issus de REF2A1 et GEL2A1.

### Exemple 3 : Préparation d'un gel G22 selon l'invention et du gel REF22 (comparatif)

Dans 40.0 g de gel REF2A (préparé dans l'exemple 2), on ajoute 5.1 g d'une solution de hyaluronate de sodium (masse moléculaire = 1.7 MDa, taux d'humidité = 4.9%) à 25 mg/ml dans une solution tampon phosphate puis on mélange manuellement pendant 5 minutes. On ajoute ensuite 8.1 g d'acide oléique et on mélange manuellement le gel à la spatule pendant 10 minutes. Soit GEL2A2 le gel ainsi obtenu.

Dans 40.0 g de gel REF2A (préparé dans l'exemple 2), on ajoute 5.1 g d'une solution de hyaluronate de sodium (masse moléculaire = 1.7 MDa, taux d'humidité = 4.9%) à 25 mg/ml dans une solution tampon phosphate puis on mélange manuellement à la spatule pendant 5 minutes. On ajoute ensuite 8.1 g de solution tampon phosphate et on mélange manuellement le gel à la spatule pendant 10 minutes. Soit REF2A2 le gel ainsi obtenu.

Les gels REF2A2 et GEL2A2 sont conditionnés en seringues de 1 ml puis stérilisés à l'autoclave à 131°C pendant 20 minutes. Soit REF22 et GEL22 (= gel selon l'invention), les gels stérilisés issus de REF2A2 et GEL2A2.

### Exemple 4 : Préparation du gel G23 (gel non pris en compte dans l'invention car G"/G' > 0.70 à 0.7 Hz) et du gel REF23 (comparatif)

Dans 30.0 g de gel REF2A (préparé dans l'exemple 2), on ajoute 15.0 g d'une solution de hyaluronate de sodium (masse moléculaire = 1.7 MDa, taux d'humidité = 4.9%) à 25 mg/ml dans une solution tampon phosphate puis on mélange manuellement à la spatule pendant 10 minutes. On ajoute ensuite 5.4 g d'acide oléique et on mélange manuellement le gel à la spatule pendant 10 minutes. Soit GEL2A3 le gel ainsi obtenu.

Dans 30.0 g de gel REF2A (préparé dans l'exemple 2), on ajoute 15.0 g d'une solution de hyaluronate de sodium (masse moléculaire = 1.7 MDa, taux d'humidité = 4.9%) à 25 mg/ml dans une solution tampon phosphate puis on mélange manuellement à la spatule pendant 10 minutes.

On ajoute ensuite 5.4 g d'une solution tampon phosphate et on mélange manuellement le gel à la spatule pendant 10 minutes. Soit REF2A3 le gel ainsi obtenu.

Les gels REF2A3 et GEL2A3 sont conditionnés en seringues de 1 ml puis stérilisés à l'autoclave à 131°C pendant 40 minutes. Soit REF23 et GEL23, les gels stérilisés issus de REF2A3 et GEL2A3.

### Exemple 5 : Préparation d'un gel G31 selon l'invention

On pèse 2.77 g d'une poudre de hyaluronate de sodium (NaHA), de masse moléculaire environ égale à 1.7 MDa, avec un taux d'humidité de 4.9%, auxquels on ajoute 27.1 g d'une solution aqueuse de NaOH à 0.25 N. L'hydratation de la poudre dure 1h30, avec une homogénéisation manuelle régulière à la spatule. 0.95 g d'une solution de 1,4-butanedioldiglycidyléther (BDDE) dilué au 1/5^{ème} dans la soude 0.25 N sont ajoutés au milieu réactionnel, suivi d'une homogénéisation mécanique de 15 minutes avant immersion dans un bain thermostaté à 50°C pendant 3h00. On ajoute une solution de tampon phosphate contenant du HCl dans le réticulat obtenu afin d'obtenir un pH=7.3 et une concentration en acide hyaluronique égale à 25 mg/ml. On laisse le gel gonfler pendant 24h à température ambiante dans cette solution et, à la fin de ce temps, on l'homogénéise manuellement à la spatule pendant 10 minutes avant de le purifier par dialyse pendant 24h en utilisant une membrane à base de cellulose (seuil de rétention = 10 000 Da) dans une solution de tampon phosphate. On mélange manuellement le gel à la spatule pendant 10 minutes. Soit REF3A le gel ainsi obtenu. La concentration en acide hyaluronique de REF3A est de 18.6 mg/ml.

On prépare ensuite des liposomes contenant de l'acide oléique. Pour cela, on utilise la technique conventionnelle d'hydratation d'un film lipidique suivie par une sonication.

Les phospholipides utilisés sont les suivants : phosphatidyl choline (PC), phosphatidyl inositol (PI) et phosphatidyl sérine (PS), en ratio molaire 1.0 / 0.5 / 0.1 respectivement.

4.3 g d'acide oléique et 27.8 g du mélange de lipides décrit ci-dessus sont dissous dans un mélange chloroforme/méthanol (50 g, ratio molaire 2.7 :1). Les solvants sont ensuite retirés par évaporation à l'évaporateur rotatif en présence d'argon, à température ambiante, suivi par un séchage des phospholipides avec acide oléique durant 18h à l'étuve sous vide, permettant alors d'obtenir un film.

Ce film de phospholipides avec acide oléique est ensuite hydraté en ajoutant une solution tampon phosphate et en agitant. Des liposomes multilamellaires sont alors formés, encapsulant l'acide oléique. Leur diamètre moyen en nombre (mesuré par diffusion dynamique de la lumière) s'étend de 450 à 1200 nm.

Une étape de sonication (sonde ultra-son baignant dans le mélange de liposomes multilamellaires) à une puissance de 10 kHz durant 10 minutes dans un bain d'eau glacée permet d'homogénéiser la solution de liposomes contenant l'acide oléique tout en réduisant le diamètre des liposomes à des valeurs moyennes en nombre s'étendant de 80 à 250 nm.

Dans 40.0 g de gel REF3A, on ajoute 13.2 g de la solution de liposomes contenant l'acide oléique préparés préalablement. On mélange manuellement le gel à la spatule pendant 10 minutes. Soit GEL3A1 le gel ainsi obtenu.

Le gel GEL3A1 est conditionné en seringues de 1 ml puis stérilisé à l'autoclave à 121°C pendant 20 minutes. Soit GEL31 (= gel selon l'invention), le gel stérilisé issu de GEL3A1.

### Exemple 6 : Préparation d'un gel G32 selon l'invention

On prépare tout d'abord des liposomes contenant de l'acide palmitique.

Pour cela, on utilise la technique conventionnelle d'hydratation d'un film lipidique suivie d'une extrusion.

Les lipides utilisés sont les suivants : phosphatidyl choline (PC), phosphatidyl glycérol (PG) et cholestérol (C), en ratio molaire 1.0 / 0.2 / 0.45 respectivement.

10.5 g d'acide palmitique (préalablement chauffé pour transformer l'acide gras sous forme solide à température ambiante en un liquide) et 30 g du mélange de lipides décrit ci-dessus sont dissous dans un mélange chloroforme/méthanol (50 g, ratio molaire 2.7 :1). Les solvants sont ensuite retirés par évaporation à l'évaporateur rotatif en présence d'argon, à température ambiante, suivi par un séchage des phospholipides avec acide palmitique durant 15h à l'étuve sous vide, permettant alors d'obtenir un film.

Le film de phospholipides et d'acide palmitique est ensuite hydraté en ajoutant une solution tampon phosphate et en agitant. Des liposomes multilamellaires sont alors formés, encapsulant l'acide palmitique. Leur diamètre moyen en nombre (mesuré par diffusion dynamique de la lumière) s'étend de 370 à 1050 nm.

Une étape d'extrusion des liposomes multilamellaires sur membrane 0.2 microns de polycarbonate (système Avanti® Polar Lipids mini-extruder) à une température de 37°C permet d'homogénéiser la solution de liposomes contenant l'acide palmitique tout en réduisant le diamètre des liposomes à des valeurs moyennes en nombre s'étendant de 40 à 210 nm.

Dans 40.0 g de gel REF3A (préparé dans l'exemple 5), on ajoute 3.8 g de la solution de liposomes contenant l'acide palmitique préparés préalablement. On mélange manuellement le gel à la spatule pendant 10 minutes. Soit GEL3A2 le gel ainsi obtenu.

Le gel GEL3A2 est conditionné en seringues de 1 ml puis stérilisé à l'autoclave à 121°C pendant 20 minutes. Soit GEL32 (= gel selon l'invention), le gel stérilisé issu de GEL3A2.

### Exemple 7 : Caractérisation des gels G1, G21, G22 selon l'invention et des gels REF1, REF21, REF22, G23 et REF23 (comparatif)

Les gels G1 (préparé à l'exemple 1), G21 (préparé à l'exemple 2) et G22 (préparé à l'exemple 3) selon l'invention sont analysés et comparés aux gels REF1 (préparé à l'exemple 1), REF21 (préparé à l'exemple 2) et REF22 (préparé à l'exemple 3) de l'art antérieur.

L'ensemble des gels est analysé en termes de force d'éjection, de propriétés rhéologiques, de cohésivité et d'aspect, et ceci après stérilisation à l'autoclave.

La cohésivité du gel à analyser est évaluée de la manière suivante : dans un flacon de 10 ml contenant 8 ml d'eau purifiée, on introduit 1 ml du gel à tester puis on agite manuellement pendant 15 secondes. En fonction des formulations analysées, on peut observer soit une très forte dissociation/fragmentation du gel dans l'eau (= niveau 1 = faible cohésivité), soit pas ou très peu de dissociation (= niveau 3 = forte cohésivité), soit un niveau de cohésivité moyen intermédiaire entre 1 et 3 (= niveau 2 = cohésivité moyenne).

L'observation de l'aspect microscopique du gel à analyser est réalisée à la binoculaire, avec un grossissement X40.

| **Gel analysé** | **Force d'éjection** | **Cohésivité** | **G' (0.7 Hz)** | **G"/G' (0.7 Hz)** | **Aspect visuel** | **Aspect microscopique** |
|---|---|---|---|---|---|---|
| G1 (selon l'invention) | 17 N | Niveau 2 | 319 Pa | 0.12 | Translucide, homogène | Observation de petites gouttelettes d'acide gras dispersées dans le gel |
| G21 (selon l'invention) | 21 N | Niveau 2 | 442 Pa | < 0.10 | Translucide, homogène | Observation de petites gouttelettes d'acide gras dispersées dans le gel |
| G22 (selon l'invention) | Non mesurée | Niveau 3 | 104 Pa | 0.51 | Translucide, homogène | Observation de petites gouttelettes d'acide gras dispersées dans le gel |
| G23 | Non mesurée | Niveau 3 | 32 Pa | 0.96 | Translucide, homogène | Observation de petites gouttelettes d'acide gras dispersées dans le gel |
| REF1 | 17 N | Niveau 2 | 327 Pa | 0.11 | Transparent | Pas de petites gouttelettes observées dans le gel |
| REF21 | 20 N | Niveau 2 | 448 Pa | < 0.10 | Transparent | Pas de petites gouttelettes observées dans le gel |
| REF22 | Non mesurée | Niveau 3 | Non mesurée | Non mesurée | Transparent | Pas de petites gouttelettes observées dans le gel |
| REF23 | Non mesurée | Niveau 3 | Non mesurée | Non mesurée | Transparent | Pas de petites gouttelettes observées dans le gel |

De manière tout à fait surprenante, on constate qu'un gel à base d'acide hyaluronique réticulé avec acide gras selon l'invention possède une force d'éjection, un niveau de cohésivité et des propriétés rhéologiques similaires à celles du gel correspondant ne contenant pas d'acide gras.

Cette caractéristique du gel selon l'invention est particulièrement avantageuse car elle permet de fournir aux praticiens des produits injectables avec acides gras possédant des propriétés similaires à celles déjà connues actuellement, et donc ayant vocation à traiter les mêmes indications, tout en visant une meilleure sécurité et performance clinique du fait de la présence d'acide gras (par exemple, meilleure capacité à créer/maintenir du volume sur le long terme).

### Exemple 8 : Préparation d'une solution d'acide hyaluronique non réticulé avec acide gras (comparatif)

Dans 5.0 g d'une solution de hyaluronate de sodium (masse moléculaire = 1.7 MDa, taux d'humidité = 4.9%) à 20 mg/ml dans une solution tampon phosphate, on ajoute 0.35 g d'acide oléique et on mélange manuellement à la spatule pendant 10 minutes. Soit SOL4A la solution ainsi obtenue.

La solution SOL4A est conditionnée en seringues de 1 ml puis stérilisée à l'autoclave à 121°C pendant 20 minutes. Soit SOL4, la solution stérilisée issue de SOL4A.

A t=0 après stérilisation, on constate que la solution SOL4, d'aspect translucide, possède quelques zones hétérogènes. Après 2 mois de stockage à température ambiante, on observe un surnageant (= phase organique d'acide gras) à la surface de la solution.

Ce type de solution (acide hyaluronique non réticulé avec acide gras), non pris en compte dans le cadre de cette invention, n'est pas compatible avec une durée de péremption de plusieurs mois du fait d'une stabilité insuffisante de l'émulsion huile dans eau.

Sans vouloir se lier à une explication, cet exemple démontre que l'acide hyaluronique doit être réticulé pour stabiliser le ou les acides gras en suspension dans l'hydrogel, ce qui permet alors d'obtenir une composition selon l'invention d'aspect visuel homogène tout en étant hétérogène au niveau microscopique.

### Exemple 9 : Evaluation de la résistance à la dégradation radicalaire et enzymatique pour le gel G21 selon l'invention et pour les gels REF21 et REF21+antioxydant.

Dans 4.9 g de gel REF21, on ajoute 4% de mannitol puis on mélange manuellement à la spatule pendant 10 minutes. Soit [REF21+antioxydant], le gel ainsi obtenu.

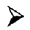 Test de dégradation radicalaire :

Dans 5 tubes à essais, on introduit respectivement (mélange manuel à la spatule de 1 min effectué pour chaque tube à essais) :
- tube 1 : 2.0 g de gel G21 (préparé à l'exemple 2)
- tube 2 : 2.0 g de gel REF21 (préparé à l'exemple 2)
- tube 3 : 2.0 g de gel G21 + 10% d'une solution de H₂O₂ (30% masse/masse)
- tube 4 : 2.0 g de gel REF21 + 10% d'une solution de H₂O₂ (30% masse/masse)
- tube 5 : 2.0 g de gel [REF21+antioxydant] + 10% d'une solution de H₂O₂ (30% m/m)

On place ensuite ces tubes à l'étuve à la température de 37°C puis, après 6 heures à cette température, on retourne l'ensemble des tubes.

On constate que les contenus des tubes 1 et 2 s'écoulent très peu et de la même manière, alors que les contenus des tubes 3 et 4 s'écoulent rapidement et de la même manière. Le contenu du tube 5, lui, avec antioxydant, a un écoulement intermédiaire entre les tubes 1/2 et les tubes 3/4.

Cette expérience montre que, lors d'une dégradation radicalaire par H₂O₂, le gel selon l'invention et le gel correspondant sans acide gras possèdent une cinétique de dégradation similaire.

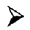 Test de dégradation enzymatique :

Dans 4 tubes à essais, on introduit respectivement (mélange manuel à la spatule de 1 min effectué pour chaque tube à essais) :
- tube 1 : 2.0 g de gel G21 (préparé à l'exemple 2)
- tube 2 : 2.0 g de gel REF21 (préparé à l'exemple 2)
- tube 3 : 2.0 g de gel G21 + 10% d'une solution de hyaluronidase (origine bovine, 200 U/ml)
- tube 4 : 2.0 g de gel REF21 + 10% d'une solution de hyaluronidase (origine bovine, 200 U/ml)

On place ensuite ces tubes à l'étuve à la température de 37°C puis, après 6 heures à cette température, on retourne l'ensemble des tubes.

On constate que les contenus des tubes 1 et 2 s'écoulent très peu et de la même manière, alors que les contenus des tubes 3 et 4 s'écoulent rapidement et de la même manière.

Cette expérience montre que, lors d'une dégradation enzymatique par les hyaluronidases, le gel selon l'invention et le gel correspondant sans acide gras possèdent une cinétique de dégradation similaire.

### Exemple 10 : Evaluation de la stabilité au cours du temps des gels G1, G21, G22 selon l'invention et du gel G23 lors d'un stockage à température ambiante (comparatif)

Les gels G1 (préparé à l'exemple 1), G21 (préparé à l'exemple 2), G22 (préparé à l'exemple 3) et G23 (préparé à l'exemple 4) sont stockés à température ambiante et observés au cours du temps.

| **Gel stocké à T°C amb** | **t = 0** | **t = 3 mois** | **t = 6 mois** |
|---|---|---|---|
| G1 (selon l'invention) | Gel translucide homogène | Gel translucide homogène | Gel translucide homogène |
| G21 (selon l'invention) | Gel translucide homogène | Gel translucide homogène | Gel translucide homogène |
| G22 (selon l'invention) | Gel translucide homogène | Gel translucide homogène | Gel translucide homogène |
| G23 | Gel translucide homogène | Gel translucide homogène | Gel translucide mais des zones hétérogènes observées |

On constate qu'après 6 mois à température ambiante les gels G1, G21 et G22 selon l'invention sont translucides et homogènes alors que le gel G23 (= gel non pris en compte dans l'invention), possédant un ratio G"/G' à 0.7 Hz supérieur à 0.70, commence à présenter des zones hétérogènes (coalescence de petites gouttelettes d'acide gras au sein du gel).

Contrairement aux gels G1, G21 et G22 selon l'invention, le gel G23 n'est pas compatible avec une durée de péremption de 24 mois à température ambiante; durée généralement requise par les praticiens (utilisateurs) pour les produits injectables à base d'acide hyaluronique réticulé.

Le ratio G"/G' à 0.7 Hz, synonyme de caractère plus ou moins élastique de la composition viscoélastique, joue donc un rôle déterminant dans la présente invention.

### Exemple 11 : Evaluation de la cinétique de libération des acides gras (comparatif)

Dans des membranes à base de cellulose (seuil de rétention = 10 000 Da), on introduit (en respectant un ratio de remplissage d'environ 3.5 g de gel par cm de membrane) :
- 10 g de gel G1 selon l'invention (préparé à l'exemple 1)
- 10 g de gel REF1 (préparé à l'exemple 1)
- 10 g de gel G22 selon l'invention (préparé à l'exemple 3)
- 10 g de gel REF22 (préparé à l'exemple 3)

Ces 4 membranes sont chacune introduites dans 500 ml de solution saline à 0.9% en NaCl et on observe les gels au cours du temps :
- à t=0, les gels G1 et G22 selon l'invention sont translucides du fait de la présence des acides gras dispersés dans les gels. Ce point est d'ailleurs confirmé par une observation à la binoculaire, avec un grossissement X40, permettant de visualiser les petites gouttelettes d'acide gras dispersées dans les gels d'acide hyaluronique réticulé. Les gels REF1 et REF22, eux, sont totalement transparents
- on effectue les mêmes observations (visuelles et à la binoculaire) après 1 jour, 3 jours, 1 semaine, 2 semaines, 3 semaines, 4 semaines, 6 semaines, 8 semaines, 24 semaines et 40 semaines. Pour chacun de ces temps, comme à t=0, les gels G1 et G22 selon l'invention sont translucides et les gels REF1 et REF22 sont transparents.

De manière tout à fait surprenante, alors qu'il est bien connu par l'homme de l'art que les molécules de tailles moyennes à petites sont rapidement libérées d'un gel à base d'acide hyaluronique réticulé (par exemple, molécule de chlorhydrate de lidocaïne libérée quasi-totalement dès les 24 premières heures - exemple 4 de WO 2010/015900), on constate que les acides gras présents dans les gels selon l'invention ne sont pas ou très peu libérés après plusieurs semaines. Cette caractéristique confère un avantage considérable aux hydrogels selon l'invention car permettant de bénéficier des avantages apportés par la synergie entre l'acide hyaluronique réticulé et les acides gras, dans les conditions de l'invention, sur de très longues périodes après implantation dans les tissus.

## Revendications

1. Composition aqueuse stérile injectable, stérilisée à la chaleur, comprenant au moins de l'acide hyaluronique réticulé, ou l'un de ses sels, et un ou plusieurs acides gras, **caractérisée en ce que** :
∘ la proportion massique en eau est supérieure à 51% de la masse totale
∘ la proportion massique en acide gras est inférieure à 45% de la masse totale
∘ les propriétés de viscoélasticité sont telles que le rapport G"/G' à 0.7 Hz est inférieur à 0.70.

2. Composition selon la revendication 1, **caractérisée en ce que** la composition est stérilisée à la chaleur humide.

3. Composition selon l'une des revendications 1 à 2, **caractérisée en ce que** l'acide hyaluronique, ou l'un de ses sels, est le hyaluronate de sodium.

4. Composition selon l'une des revendications 1 à 3, **caractérisée en ce que** l'acide hyaluronique, ou l'un de ses sels, est réticulé en utilisant un réticulant bi ou poly fonctionnel, en particulier le 1,4-butanedioldiglycidyléther (BDDE).

5. Composition selon l'une des revendications 1 à 4, **caractérisée en ce que** la concentration totale en acide hyaluronique, ou l'un de ses sels, est comprise entre 9 et 30 mg/ml.

6. Composition selon l'une des revendications 1 à 5, **caractérisée en ce que** la proportion massique en eau est supérieure à 80% de la masse totale, en particulier supérieure à 85% de la masse totale.

7. Composition selon l'une des revendications 1 à 6, **caractérisée en ce que** la proportion massique en acide gras est inférieure à 25% de la masse totale, en particulier inférieure à 10% de la masse totale.

8. Composition selon l'une des revendications 1 à 7, **caractérisée en ce que** le ou les acides gras sont choisis parmi l'acide butyrique, l'acide caproïque, l'acide caprylique, l'acide laurique, l'acide myristique, l'acide palmitique, l'acide stéarique, l'acide palmitoléique, l'acide oléique, l'acide linoléique, l'acide alpha-linoléique et l'acide arachidonique.

9. Composition selon l'une des revendications 1 à 8, **caractérisée en ce que** la concentration totale en acide gras est supérieure à 1 mg/ml.

10. Composition selon l'une des revendications 1 à 9, **caractérisée en ce que** la composition contient des liposomes ou des niosomes.

11. Composition selon l'une des revendications 1 à 10, **caractérisée en ce que** au moins une partie des acides gras est contenue dans des liposomes ou des niosomes.

12. Procédé de préparation d'une composition aqueuse stérile injectable comprenant au moins de l'acide hyaluronique réticulé, ou l'un de ses sels, et un ou plusieurs acides gras, **caractérisé en ce qu'**il comprend au moins les étapes successives suivantes :
a) préparation d'un hydrogel à base d'acide hyaluronique réticulé, ou l'un de ses sels
b) ajout du ou des acides gras dans l'hydrogel avec opération de mélange
c) stérilisation à l'autoclave.

13. Procédé de préparation d'une composition aqueuse stérile injectable comprenant au moins de l'acide hyaluronique réticulé, ou l'un de ses sels, et un ou plusieurs acides gras, selon la revendication 12, **caractérisé en ce qu'**il comprend au moins les étapes successives suivantes :
- préparation d'une solution aqueuse d'acide hyaluronique
- réticulation de l'acide hyaluronique
- purification de l'hydrogel
- ajout d'un ou plusieurs acides gras avec opération de mélange, par exemple par ajout dans l'hydrogel de liposomes ou de niosomes contenant un ou plusieurs acides gras
- conditionnement en seringues
- stérilisation à l'autoclave.

14. Utilisation de la composition selon l'une des revendications 1 à 11 pour des applications esthétiques.

15. Composition selon l'une des revendications 1 à 11, pour utilisation dans le cadre d'un traitement thérapeutique.

## Patentansprüche

1. Injizierbare sterile wässrige, mit Wärme sterilisierte Zusammensetzung, umfassend mindestens vernetzte Hyaluronsäure oder eines ihrer Salze und eine oder mehrere Fettsäuren, **dadurch gekennzeichnet, dass**:
∘ der Massenanteil an Wasser größer als 51% der Gesamtmasse ist
∘ der Massenanteil an Fettsäure kleiner als 45% der Gesamtmasse ist
∘ die Viskoelastizitätseigenschaften derart sind, dass das Verhältnis G"/G' bei 0,7 Hz kleiner als 0,70 ist.

2. Zusammensetzung nach Anspruch 1, **dadurch gekennzeichnet, dass** die Zusammensetzung bei feuchter Wärme sterilisiert ist.

3. Zusammensetzung nach einem der Ansprüche 1 bis 2, **dadurch gekennzeichnet, dass** die Hyaluronsäure oder eines ihrer Salze das Natriumhyaluronat ist.

4. Zusammensetzung nach einem der Ansprüche 1 bis 3, **dadurch gekennzeichnet, dass** die Hyaluronsäure oder eines ihrer Salze bei Verwendung eines bi- oder polyfunktionalen Vernetzungsmittels, insbesondere des 1,4-Butandioldiglycidylethers (BDDE), vernetzt wird.

5. Zusammensetzung nach einem der Ansprüche 1 bis 4, **dadurch gekennzeichnet, dass** die Gesamtkonzentration an Hyaluronsäure oder von einem ihrer Salze zwischen 9 und 30 mg/ml liegt.

6. Zusammensetzung nach einem der Ansprüche 1 bis 5, **dadurch gekennzeichnet, dass** der Massenanteil an Wasser größer als 80% der Gesamtmasse, insbesondere größer als 85% der Gesamtmasse ist.

7. Zusammensetzung nach einem der Ansprüche 1 bis 6, **dadurch gekennzeichnet, dass** der Massenanteil an Fettsäure kleiner als 25% der Gesamtmasse, insbesondere kleiner als 10% der Gesamtmasse ist.

8. Zusammensetzung nach einem der Ansprüche 1 bis 7, **dadurch gekennzeichnet, dass** die Fettsäure(n) aus der Buttersäure, der Capronsäure, der Caprylsäure, der Laurinsäure, der Myristinsäure, der Palmitinsäure, der Stearinsäure, der Palmitoleinsäure, der Oleinsäure, der Linolsäure, der Alpha-Linolsäure und der Arachidonsäure ausgewählt sind.

9. Zusammensetzung nach einem der Ansprüche 1 bis 8, **dadurch gekennzeichnet, dass** die Gesamtkonzentration an Fettsäure größer als 1 mg/ml ist.

10. Zusammensetzung nach einem der Ansprüche 1 bis 9, **dadurch gekennzeichnet, dass** die Zusammensetzung Liposomen oder Niosomen enthält.

11. Zusammensetzung nach einem der Ansprüche 1 bis 10, **dadurch gekennzeichnet, dass** mindestens ein Teil der Fettsäuren in Liposomen oder Niosomen enthalten ist.

12. Verfahren zur Herstellung einer injizierbaren sterilen wässrigen Zusammensetzung, die mindestens vernetzte Hyaluronsäure oder eines ihrer Salze umfasst und eine oder mehrere Fettsäuren, **dadurch gekennzeichnet, dass** es mindestens die folgenden aufeinanderfolgenden Schritte umfasst:
a) Herstellen eines Hydrogels auf der Basis von vernetzter Hyaluronsäure oder einem ihrer Salze
b) Hinzufügen der Fettsäure(n) in das Hydrogel mit Mischvorgang
c) Sterilisieren im Autoklav.

13. Verfahren zur Herstellung einer injizierbaren sterilen wässrigen Zusammensetzung, die mindestens vernetzte Hyaluronsäure oder eines ihrer Salze umfasst und eine oder mehrere Fettsäuren, nach Anspruch 12, **dadurch gekennzeichnet, dass** es mindestens die folgenden aufeinanderfolgenden Schritte umfasst:
- Herstellen einer wässrigen Hyaluronsäurelösung
- Vernetzen der Hyaluronsäure
- Reinigen des Hydrogels
- Hinzufügen von einer oder mehreren Fettsäuren mit Mischvorgang, beispielsweise durch Hinzufügen in das Hydrogel von Liposomen oder von Niosomen, die eine oder mehrere Fettsäuren enthalten
- Verpacken in Spritzen
- Sterilisieren im Autoklav.

14. Verwendung der Zusammensetzung nach einem der Ansprüche 1 bis 11 für ästhetische Anwendungen.

15. Zusammensetzung nach einem der Ansprüche 1 bis 11 für Verwendung im Rahmen einer therapeutischen Behandlung.

## Claims

1. A sterile injectable aqueous composition, sterilized by heat, comprising at least cross-linked hyaluronic acid, or a salt thereof, and one or more fatty acids, **characterized in that**:
∘ the mass proportion of water is greater than 51% of the total mass
∘ the mass proportion of fatty acid is less than 45% of the total mass
∘ the viscoelastic properties are such that the ratio G"/G' at 0.7 Hz is less than 0.70.

2. The composition according to claim 1, **characterized in that** the composition is sterilized by moist heat.

3. The composition according to one of claims 1 to 2, **characterized in that** the hyaluronic acid, or a salt thereof, is sodium hyaluronate.

4. The composition according to one of claims 1 to 3, **characterized in that** the hyaluronic acid, or a salt thereof, is cross-linked using a bi- or poly-functional cross-linker, in particular 1,4-butanediol diglycidyl ether (BDDE).

5. The composition according to one of claims 1 to 4, **characterized in that** the total concentration of hyaluronic acid, or a salt thereof, is between 9 and 30 mg/ml.

6. The composition according to one of claims 1 to 5, **characterized in that** the mass proportion of water is greater than 80% of the total mass, in particular greater than 85% of the total mass.

7. The composition according to one of claims 1 to 6, **characterized in that** the mass proportion of fatty acid is less than 25% of the total mass, in particular less than 10% of the total mass.

8. The composition according to one of claims 1 to 7, **characterized in that** the fatty acid(s) is/are selected from butyric acid, caproic acid, caprylic acid, lauric acid, myristic acid, palmitic acid, stearic acid, palmitoleic acid, oleic acid, linoleic acid, alpha-linoleic acid and arachidonic acid.

9. The composition according to one of claims 1 to 8, **characterized in that** the total concentration of fatty acid is greater than 1 mg/ml.

10. The composition according to one of claims 1 to 9, **characterized in that** the composition contains liposomes or niosomes.

11. The composition according to one of claims 1 to 10, **characterized in that** at least a portion of the fatty acids is contained in liposomes or niosomes.

12. A method for preparing a sterile injectable aqueous sterile composition comprising at least cross-linked hyaluronic acid, or a salt thereof, and one or more fatty acids, **characterized in that** it comprises at least the following successive steps:
a) preparation of a hydrogel based on cross-linked hyaluronic acid, or a salt thereof
b) addition of the fatty acid(s) to the hydrogel with a mixing operation
c) autoclave sterilization.

13. The method for preparing a sterile injectable aqueous composition comprising at least cross-linked hyaluronic acid, or a salt thereof, and one or more fatty acids, according to claim 12, **characterized in that** it comprises at least the following successive steps:
- preparation of an aqueous solution of hyaluronic acid
- cross-linking of the hyaluronic acid
- purification of the hydrogel
- addition of one or more fatty acids with a mixing operation, for example by adding to the hydrogel liposomes or niosomes containing one or more fatty acids
- packaging in syringes
- autoclave sterilization.

14. Use of the composition according to one of claims 1 to 11 for aesthetic applications.

15. The composition according to one of claims 1 to 11, for use in therapeutic treatment.
